(19) Europäisches Patentamt
European Patent Office
Office européen des brevets

(11) **EP 0 985 416 A2**

(12) **EUROPÄISCHE PATENTANMELDUNG**

(43) Veröffentlichungstag:
15.03.2000 Patentblatt 2000/11

(51) Int. Cl.[7]: **A61K 35/78**, A61K 41/00,
H05B 6/80, H05B 6/62

(21) Anmeldenummer: 99116562.2

(22) Anmeldetag: 24.08.1999

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **24.08.1998 DE 19838377**

(71) Anmelder:
**Fiegert-Seibt, Edda, Dr.
81297 München (DE)**

(72) Erfinder:
**Fiegert-Seibt, Edda, Dr.
81297 München (DE)**

(74) Vertreter:
**Feldkamp, Rainer, Dipl.-Ing. et al
Garmischer Strasse 4
80339 München (DE)**

(54) **Verfahren und Vorrichtung zur Extraktherstellung aus biologischem Material**

(57) Die Erfindung betrifft ein Verfahren zur parametergesteuerten Herstellung von modifizierten Extrakten biologischer Materialien unter Einwirken von Mikrowellen und gleichzeitiger Bestrahlung durch eine Strahlungsquelle sowie fakultativer Begasung mit reaktiven Gasen oder Inertgasen. Weiterhin betrifft die Erfindung die mit Hilfe des erfindungsgemäßen Verfahrens hergestellten Extrakte und deren Verwendung in Arzneimitteln, kosmetischen und Körperpflegeprodukten sowie in Lebensmittelprodukten und eine Vorrichtung zur Durchführung des Verfahrens.

EP 0 985 416 A2

**Beschreibung**

Die Erfindung betrifft ein Verfahren zur parametergesteuerten Herstellung von modifizierten Extrakten biologischer Materialien unter Einwirkung von Mikrowellen und gleichzeitiger Bestrahlung durch eine Strahlungsquelle. Weiterhin betrifft die Erfindung die mit Hilfe des erfindungsgemäßen Verfahrens hergestellten Extrakte und deren Verwendung in Arzneimitteln, kosmetischen und Körperpflegeprodukten und Lebensmittelprodukten sowie eine Vorrichtung zur Durchführung der Verfahren.

Die Gewinnung von Auszügen (Extrakten) aus biologischem Material erfolgt üblicherweise durch Mazeration oder durch Strömung (Perkolation, Wirbelextraktion, Soxhletextraktion) des ganzen oder zerkleinerten, frischen oder getrockneten pflanzlichen Materials mit (während der Extraktion) flüssigen oder gasförmigen Lösungsmitteln oder durch Destillation der flüchtigen Bestandteile mit oder ohne Wasserdampf.

Bei der konventionellen Extraktion sollen die Pflanzeninhaltsstoffe in der Regel möglichst ohne unerwünschte chemische Veränderung und in möglichst hoher Konzentration im jeweiligen Lösungsmittel gelöst werden, wie es zum Beispiel bei den in der Homöopathie üblichen Urtinkturen der Fall ist. Häufig werden diese Extrakte als "Gesamtextrakte" bezeichnet.

Modernere Extraktionsverfahren streben hingegen das selektive Lösen und Anreichern bestimmter Stoffgruppen an (zum Beispiel valepotriathaltige Spezialextrakte aus Baldrian). Diese Zubereitungen, die häufig im Anschluß an die Extraktion noch weiter chemisch oder physikalisch behandelt werden, werden meistens als "Spezialextrakte" bezeichnet.

Alle bisher bekannten Verfahren sind zeit- und kostenaufwendig und führen sehr häufig aufgrund der langen Exposition der Pflanzeninhaltsstoffe gegenüber erhöhten Temperaturen und Luftsauerstoff zu unkontrollierten sekundären chemischen Veränderungen. So erfahren beispielsweise ätherische Öle, die wie üblich durch Wasserdampfdestillation gewonnen wurden, chemische Umwandlungen (in Kamillenöl wird farbloses Matricin zu blauem Chamazulen umgewandelt).

Die Herstellung von Ölextrakten als Sonderform erfolgt entweder traditionell durch wochenlange Mazeration des Pflanzenmaterials im Öl oder nach einer Lösungsmittelextraktion und Abdampfen des Lösungsmittels durch anschließende Lösung des möglichst lösungsmittelfreien Dickextrakts im Öl.

Der erste Weg ist zeitaufwendig und damit kostenintensiv, auch die zweite Variante (Abdampfen des Lösungsmittels) zieht hohe Kosten nach sich. Zudem ist die restlose Entfernung des Lösungsmittels in der Regel nahezu unmöglich, was eine potentielle Belastung des Extraktes und der Umwelt zur Folge hat.

Bei beiden Verfahren kommt es ebenfalls zu unkontrolliert ablaufenden sekundären Veränderungen der Extraktivstoffe, die entweder erwünscht (Beispiel Johanniskraut: Hypericinbildung) oder unerwünscht sind (Beispiel Knoblauch: Allicinbildung).

Die Herstellung von Spezialextrakten macht neben dem eigentlichen Extraktionsverfahren, wie oben angesprochen, weiterhin eine zusätzliche Behandlung des die Inhaltsstoffe enthaltenden Extraktionsmittels nötig. Beispielsweise ist es bei der Herstellung von standardisierten Johanniskrautextrakten während der Ölextraktion üblich, das mit der Droge versetzte Öl über eine Zeitspanne von mehreren Wochen dem Sonnenlicht auszusetzen, wodurch der eigentliche Hauptinhaltsstoff erst gebildet wird (Hypericin). Auch dieser Weg ist zeitaufwendig und damit sehr kostenintensiv. Zudem besteht die Gefahr, daß das Öl bei dieser Vorgehensweise ranzig wird und somit verworfen werden muß.

Ein Ersatz bisher üblicher Verfahren zur Extraktion von Naturprodukten durch mikrowellenunterstützte Extraktion ist bereits seit längerer Zeit bekannt.

Die US-Patentschrift 5 338 557 mit dem Titel "Microwave extraction of volatile oils" offenbart ein Verfahren zur Extraktion löslicher Produkte aus biologischem Material, einschließlich ätherischer Öle, durch Aussetzen der zerkleinerten, mit einem nicht-wäßrigen Lösungsmittel versetzten Ausgangsmaterialien gegenüber Mikrowellenenergie. Dabei sollen die flüchtigen Bestandteile gleich nach ihrer Freisetzung durch das Extraktionsmittel abgekühlt werden, worauf die extrahierte Phase vom restlichen Material getrennt wird.

Pare et al. offenbaren in der US-Patentschrift 5 002 784 mit dem Titel "Microwave-assisted natural products extraction" ein Verfahren zur Mikrowellenextraktion von Naturprodukten, bei dem das zerkleinerte Ausgangsmaterial mit einem Extraktionsmittel versetzt und nachfolgend einer Mikrowellenbehandlung ausgesetzt wird, wobei das zu behandelnde Ausgangsmaterial zumindest 30 % Extraktionsmittel enthält und vorzugsweise 40 bis 90 % (G/G) Feuchtigkeit enthält.

Die US-Patentschrift 5 377 426 mit dem Titel "Microwave-assisted generation of volatiles, of supercritical fluid, and apparatus therefor" beschreibt ein Verfahren, bei dem verflüchtigbare Bestandteile durch Einbringen des Ausgangsmaterials in einen selektiv durchlässigen Behälter, mit nachfolgender Mikrowellenbehandlung gewonnen werden.

Die DE 196 05 650 beschreibt ein Extraktionsverfahren von Inhaltsstoffen aus biologischen Materialien sowie zur Behandlung von Saat- und Vermehrungsgut, bei dem gleichzeitig eine Keimabtötung in Kombination mit der gleichzeitigen Anwesenheit von Wasserdampf oder Wasserdampfnebel im Behandlungsbereich durchgeführt wird. Dieses Verfahren erfolgt unter Vergleichmäßigung des Energiefelds der elektromagnetischen bzw. Mikrowellenschwingungen, wobei die Kühlgrenztemperatur des Gutes in der bestehenden Atmosphäre nicht wesentlich unterschritten wird.

Jedoch offenbart keine der vorhergehenden Druckschriften ein Verfahren, bei dem alle Arten von Inhaltsstoffen (zum Beispiel frische oder getrocknete Pflanzenteile) aus Ausgangsmaterialien biologischer Herkunft mit polaren und/oder unpolaren Lösungsmitteln gleichzeitig extrahiert und modifiziert werden können.

Die vorliegende Erfindung soll folglich ein Verfahren, eine Vorrichtung zur Durchführung dieses Verfahrens und durch dieses Verfahren hergestellte Erzeugnisse bereitstellen, durch die mit einer gezielten Abfolge von Reaktionsschritten in kürzerer Zeit, mit geringeren Kosten, ohne die Gefahr der Bildung unerwünschter Nebenprodukte, ohne Belastung des Extraktionsgutes mit Lösungsmittelrückständen bzw. ohne Belastung der Umwelt, Extrakte mit einem speziellen, qualitativ wie quantitativ bisherigen Verfahren überlegenen Inhaltsstoffmuster erzeugt werden können. Dabei sollen alle Arten von biologischen Ausgangsmaterialien und alle Arten von Lösungsmitteln eingesetzt werden können.

Die vorliegende Erfindung beschreibt weiterhin die Verwendung der nach dem erfindungsgemäßen Verfahren hergestellten Extrakte in Arzneimitteln und Lebensmittelprodukten.

Gelöst werden diese Aufgaben durch die in den Patentansprüchen 1, 12, 13 und 14 angegebenen Merkmale.

Vorteilhafte Ausgestaltungen und Weiterbildungen ergeben sich aus den Unteransprüchen.

Es zeigte sich, daß bei geeigneter Gerätekonstellation und gesteuerter Dosierung der Mikrowellenenergie sowie dem Einsatz zusätzlicher Prozeßsteuerungselemente wie Bestrahlung, Begasung, Druck und Temperatur, in kürzester Zeit hochwertige Extrakte aus frischer, vorgetrockneter oder getrockneter biologischer Matrix hergestellt werden können.

Es hat sich insbesondere gezeigt, daß durch gleichzeitige Behandlung des Extraktionsgutes mit Mikrowellen und UV-Bestrahlung wesentlich effizientere Extrakte gewonnen werden konnten, als durch Extraktion mit Mikrowellen und nachfolgender Bestrahlung bzw. andere nachfolgende Behandlungsschritte.

Weiterhin hat sich herausgestellt, daß durch gezielten Einsatz verschiedener Gase, d.h. beispielsweise durch Unterbinden der Einwirkung von Sauerstoff durch Inertbegasung oder durch Einwirkung reaktiver Gase gezielt Modifikationen am Extraktionsgut vorgenommen werden können.

Zudem wird durch eine gezielte Steuerung der Temperatur im Extraktionsbehälter sichergestellt, daß thermolabile Inhaltsstoffe durch die Behandlung mit Mikrowellen nicht über ihren Zersetzungspunkt hinaus erhitzt und somit zerstört werden. Dies kann gemäß einer bevorzugten Ausführungsform auch dadurch unterstützt werden, daß die Mikrowellenbehandlung stufenweise erfolgt, so daß eine übermäßige Erwärmung des Extraktionsgutes verhindert werden kann. Jedoch sollte aus Gründen der Zeitersparnis auch bei einer solchen Vorgehensweise die Bestrahlung durch die künstliche Strahlungsquelle ununterbrochen erfolgen.

Als Ausgangsmaterial für das vorliegende Verfahren zur Herstellung von modifizierten Extrakten können getrocknete oder frische pflanzliche Materialien eingesetzt werden, wobei auch vorgetrocknete Drogen mit einem reduzierten, jedoch über der Restfeuchte des getrockneten Materials liegenden Feuchtegehalt verwendet werden können. Das Material wird in einem ersten Schritt zerkleinert, so daß die nachfolgende Extraktion aufgrund der erhöhten Oberfläche des Gesamtmaterials effektiver ablaufen kann. Der Zerkleinerungsgrad ist dabei von der Art des eingesetzten Materials abhängig und ist oftmals durch den Einsatz marktüblicher Produkte vorgegeben.

Im Anschluß an die Zerkleinerung wird das Ausgangsmaterial mit polaren und/oder apolaren Lösungsmitteln versetzt, wobei die chemische Natur des zu extrahierenden Inhaltsstoffes das Mischungsverhältnis vorgibt. Ausnahmsweise kann das Zusetzen eines Lösungsmittels dann entfallen, wenn eine Frischdroge (wie beispielsweise die frische Wurzel von Harpagophytum procumbens) einen sehr hohen Feuchtigkeitsgehalt aufweist. Dann dient die in der Pflanze vorhandene Flüssigkeit als Extraktionsmittel. Der Ansatz wird sodann in den Extraktionsbehälter eingefüllt, der abhängig von der Art des Lösungsmittels offen oder geschlossen sein kann. Die Verwendung geschlossener Extraktionsbehälter hat sich beispielsweise beim Einsatz hydrophiler bzw. polarer Lösungsmittel als vorteilhaft erwiesen, da ein Verkochen des Ansatzes bei der Behandlung mit Mikrowellen durch den im Extraktionsbehälter entstehenden Druck verhindert wird. Gemäß einer bevorzugten Ausführungsform der Erfindung beträgt die Dichte der Mikrowellenstrahlung während der Behandlung maximal 48 W/l, vorzugsweise jedoch 12 W/l, wodurch die thermische Belastung des Extraktionsgutes verringert wird. Dies entspricht beispielsweise bei einer handelsüblichen Mikrowelle mit einem Innenvolumen von 42 l einer Leistung der Strahlungsquelle von ca. maximal 2000 W, vorzugsweise ca. 500 W. Die exakte Mikrowellenleistung und die Zeitdauer der Behandlung ist von der Schichtdicke des Extraktionsgutes und von dessen chemischen und physikalischen Eigenschaften abhängig.

Gleichzeitig mit der Mikrowellenbehandlung erfolgt eine die Inhaltsstoffe modifizierende Bestrahlung durch eine künstliche Strahlungsquelle, was, wie in den beigefügten Beispielen ausgeführt wird, eine erhöhte Ausbeute des gewünschten Endprodukts zur Folge hat.

Bei einer besonders bevorzugten Ausführungsform ist die modifizierende Strahlungsquelle eine UV-Lichtquelle.

Es können jedoch, abhängig von der erwünschten Modifikation der Inhaltsstoffe auch andere Strahlungsquellen, wie beispielsweise β- oder γ- Strahlenquellen eingesetzt werden.

Als apolare Extraktionsmittel können mikrowellentransparente Mittel wie Hexan, Dichlormethan, Öle, Wachse und Fette auf pflanzlicher oder Kohlenwasserstoff-Grundlage eingesetzt werden. Dabei kann, wie auch bei polaren Lösungsmitteln, eine Begasung mit Inertgas erfolgen, wobei durch die äußerst geringe Zeitdauer der Behandlung und durch den Sauerstoffausschluß unerwünschte Zersetzungsreaktionen, wie zum Beispiel Hydrolysen, sehr stark unterdrückt werden. Als polare Extraktionsmittel werden Wasser, Alkohole, beispielsweise Glyzerin, Ethanol, Methanol, Propylenglykol, Polyethylenglykol bzw. deren Mischungen besonders bevorzugt.

Das erfindungsgemäße Verfahren ist in der bisher beschriebenen Form vor allem dazu geeignet, die genuinen Inhaltsstoffgemische möglichst ohne qualitative und quantitative Veränderungen in das Lösungsmittel zu überführen, wobei durch gezielte, gesteuerte Veränderung einzelner Parameter, wie zum Beispiel der Energiezufuhr, der Temperatur (die Temperaturen werden bei thermolabilen Inhaltsstoffen in jedem Fall unter ihrem Zersetzungspunkt gehalten) sowie durch zusätzliche oberflächliche oder sprudelnde Begasung mit verschiedenen reaktiven oder inerten Gasen sowie durch Bestrahlung mit verschiedenen Strahlungsquellen, beispielsweise mit UV-Licht, das Verfahren so durchgeführt wird, daß bereits während des Extraktionsprozesses gewünschte chemische oder physikalische Reaktionen ablaufen können.

Die nach dem Verfahren der vorliegenden Erfindung hergestellten Extraktionsmischungen werden nach Abschluß der Extraktion durch bekannte Verfahren von den Rückständen des pflanzlichen Materials getrennt.

Die nach dem erfindungsgemäßen Verfahren hergestellten Extrakte sind zur Verwendung in Arzneimitteln, in kosmetischen- und Körperpflegeprodukten sowie Lebensmittelprodukten vorgesehen. Sie können sowohl zur inneren wie auch zur äußerlichen Anwendung -je nach den Verwendungsmöglichkeiten der Inhaltestoffe- eingesetzt werden.

Die Vorrichtung zur Durchführung besteht aus einer Mikrowellenanlage in der das Extraktionsgut einem geeigneten Gas ausgesetzt werden kann und einem Extraktionsgefäß, das bei Bedarf (z.B. hydrophile Extraktionsmittel) druckfest verschlossen werden kann. Die Vorrichtung besteht weiterhin aus einer Strahlungsquelle, die sowohl außerhalb als auch innerhalb des Reaktionsgefäßes angeordnet sein kann. Zuletzt umfaßt die Vorrichtung einen oder mehrere Thermomeßfühler und eine geeignete Einrichtung zur Anhebung oder Absenkung der Temperatur im Inneren der Mikrowellenanlage.

Das erfindungsgemäße Verfahren betrifft beispielsweise die Erzeugung genuiner oder hypericinreicher alkoholisch-wäßriger Johanniskrautextrakte bzw. genuinen oder hypericinreichen Johanniskrautköls durch direkte Ölextraktion, unter zusätzlicher UV-Bestrahlung. Weiterhin können hypericinreicher Harungaextrakt, genuiner oder valepotriatfreier Baldrianextrakt durch gezielte Umwandlung der Valepotriate, geruchneutraler Knoblauchextrakt sowie grüner oder blauer Kamillenextrakt hergestellt werden.

Beispiele für die Durchführung des erfindungsgemäßen Verfahrens sind nachfolgend angegeben, wobei die Erfindung am Beispiel Johanniskraut- und Harungaextraktion dargestellt werden soll.

Die Beispiele beschreiben erfindungsgemäße Verfahren, bei denen pflanzliches Ausgangsmaterial mit unterschiedlichen Extraktionsmitteln, unterschiedlicher Behandlungsdauer und Behandlungsstärke sowie - zu Vergleichszwecken - mit und ohne UV-Bestrahlung behandelt wird.

Die Ergebnisse des photometrischen Vergleichs der in den Beispielen erzeugten Extrakte mit Auswertung der UV-VIS-Spektren, sowie die Originalspektren sind den Beispielen beigefügt.

In den Abbildungen zeigt:

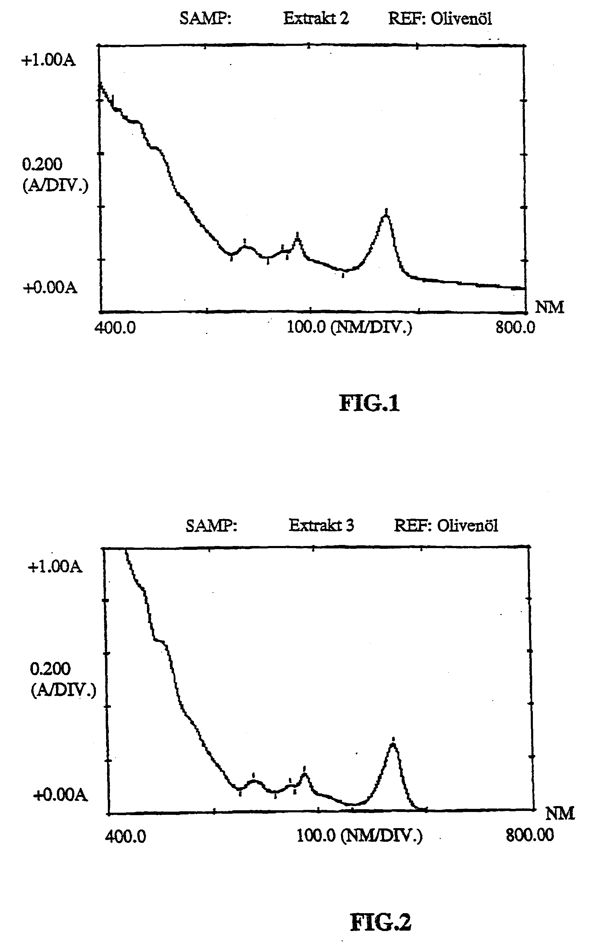

Figur 1 die UV-VIS-Spektren von Extrakt 2 aus Beispiel 1b,

Figur 2 die UV-VIS-Spektren von Extrakt 3 aus Beispiel 1b,

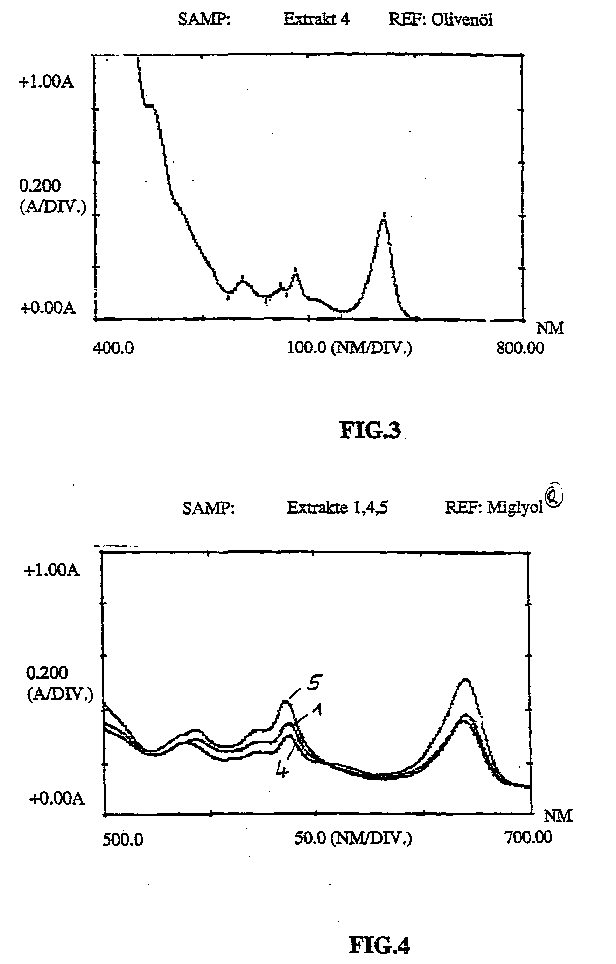

Figur 3 die UV-VIS-Spektren von Extrakt 4 aus Beispiel 1b,

Figur 4 die UV-VIS-Spektren der Extrakte 1,4 und 5 im Vergleich,

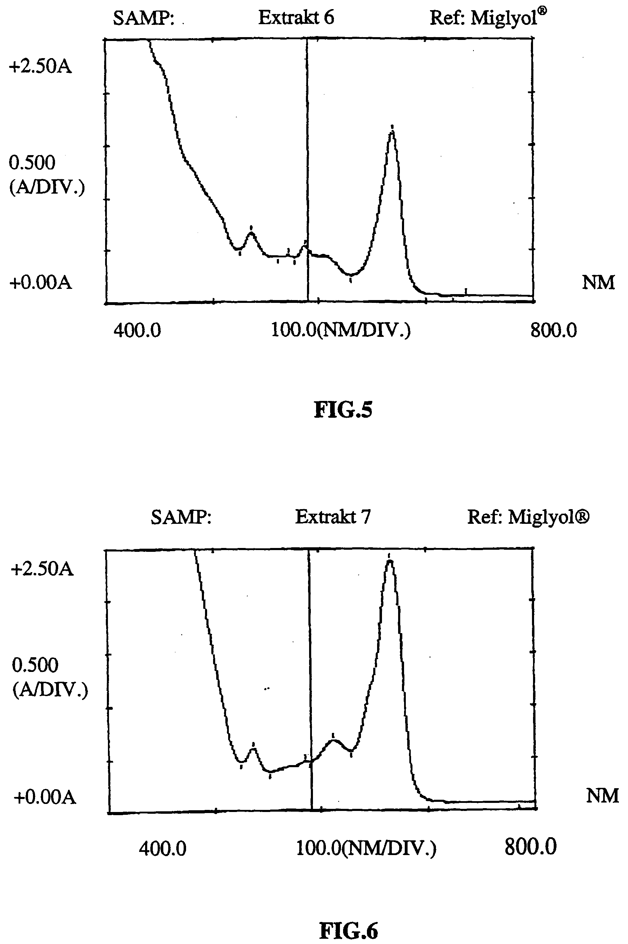

Figur 5 die UV-VIS-Spektren von Extrakt 6 aus Beispiel 3,

Figur 6 die UV-VIS-Spektren von Extrakt 7 aus Beispiel 4,

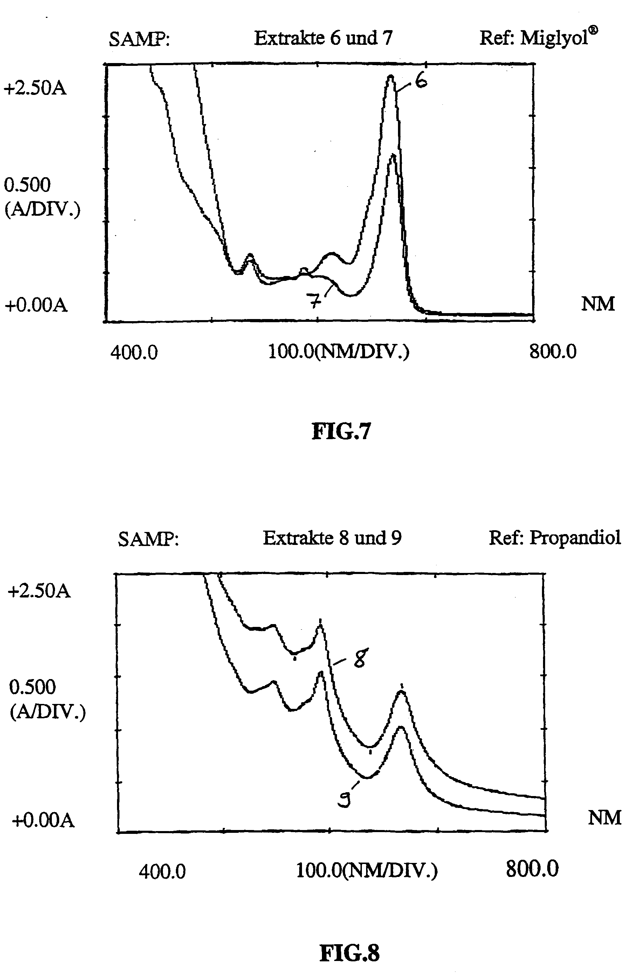

Figur 7 die UV-Vis-Spektren der Extrakte 6 und 7 im Vergleich,

Figur 8 die UV-VIS-Spektren der Extrakte 8 und 9 im Vergleich.

BEISPIEL 1 a

Die oberen Sproßteile von frisch geerntetem Johanniskraut (Hypericum perforatum) wurden vor der Extraktion in einer Moulinex®-Zerkleinerungsvorrichtung zerkleinert. Die Droge enthielt einen Feuchtigkeitsgehalt von ca. 78 %. Der Droge wurde durch Trocknung 25% der Feuchte entzogen. 20 g zerkleinertes Pflanzenmaterial wurden mit 300 ml Olivenöl versetzt und in einem Ethos 1600® Mikrowellengerät der Firma MLS-GmbH, mit simultaner UV-Bestrahlung neben dem Reaktionsgefäß, Mikrowellen ausgesetzt. Die Mikrowellenbehandlung erfolgte in drei Stufen zu je einer Minute, wobei die Mikrowellenleistung des Geräts 1000 W betrug. Das gesamte Verfahren wurde unter Stickstoffbegasung vorgenommen. Der Extrakt (Extrakt 1) zeigte eine intensive Rotfärbung, was auf einen hohen Hypericingehalt hinweist.

Eine photometrische Auswertung der UV-VIS-Spektren ergab eine Extinktion bei 586-590 nm von 0,350, was einem Hypericingehalt von 0,4023 mg% Hypericin entspricht. Die Berechnung des Hypericingehalts erfolgte mit folgender Formel:

$$\text{mg \% Hypericin} = E / 870 \times 1000$$

BEISPIEL 1 b

Beispiel 1b wurde wie Beispiel 1a durchgeführt, außer daß das Verfahren ohne UV-Bestrahlung durchgeführt wurde. Es ergab sich bei 586-590 nm eine Extinktion von 0,174 und damit ein rechnerischer Hypericingehalt von nur 0,2001 mg%. Der Extrakt (Extrakt 2) selbst zeigte im Gegensatz zu dem in Beispiel 1 hergestellten Extrakt nur leichte Rotfärbung. Um zu klären, ob der gemäß Beispiel 1b erhaltene Extrakt auch bei nachträglicher Bestrahlung höhere Hypericingehalte aufweisen würde, wurde ein Teil des Überstandes von Extrakt 2 in ein separates Glasgefäß überführt und in das Reaktionsgefäß eingesetzt. Es folgte dieselbe Behandlung wie in Beispiel 1a (Extrakt 3).

Der Rest von Extrakt 2, der das gesamte Pflanzenmaterial enthielt, wurde derselben Behandlung unterworfen (Extrakt 4).

Beide Extrakte 3 und 4 zeigten jedoch auch nach dieser Behandlung verglichen mit Extrakt 1 wesentlich niedrigere Hypericingehalte von 0,16 mg% bzw. 0,34 mg%.

Diese Ergebnisse zeigen deutlich, daß die Bestrahlung mit UV-Licht während der Extraktion wichtig ist, da auch durch Nachbehandlung des Extraktes mit einer entsprechenden Strahlung keine angemessenen Ergebnisse erzielt werden können.

BEISPIEL 2

20 g zerkleinerte Frischdroge (siehe Beispiel 1a) wurden vorgetrocknet und der Feuchtigkeitsgehalt dadurch auf ungefähr 53 % erniedrigt. Anschließend wurde das Ausgangsmaterial mit 300 ml Miglyol 812® Ph.Eur.97 (Caelo) versetzt:

Es folgte eine vierstufige Behandlung zu jeweils einer Minute mit einer Mikrowellenleistung des Geräts aus Beispiel 1 von 500 W unter Rühren (Magnetrührer). Bereits nach der dritten Stufe zeigte sich eine stark rot gefärbte Lösung, was auf einen hohen Hypericingehalt hinweist. Die Auswertung UV-VIS-Spektren ergab einen E 586-590 von 0,431 entsprechend einem Hypericingehalt von 0,49 % (Extrakt 5).

Die Beispiele 1a und 2 zeigen größenordnungsmäßig ähnliche Ergebnisse: Das UV-Licht ist bei dieser Extraktion von entscheidender Bedeutung. Beispiel 1b zeigt, daß ohne UV-Licht nur wenig Hypericin gelöst wird. Eine anschließende Beleuchtung der Lösung ist wenig effektiv, während hingegen sich bei Belichtung noch weiteres Hypericin in der Droge selbst bilden kann (Extrakt 4).

BEISPIEL 3

Frisches, blühendes Johanniskraut (Feuchtegehalt 67%) wurde ohne vorangehende Trocknung direkt zerkleinert (s. Beispiel 1) und mit Miglyol 812® im Verhältnis 1:15, bezogen auf das Trockengewicht der Droge, versetzt. Die Mikrowellenbehandlung erfolgte bei max. 1000 W ununterbrochen 15 min. lang unter UV-Bestrahlung. Die Extinktion bei 590 nm betrug 0,497, entsprechend 0,5713 mg% Hypericin (Extrakt 6).

BEISPIEL 4

Beispiel 5 wird wie Beispiel 4 durchgeführt, jedoch wird getrocknetes Johanniskraut (Feuchtegehalt 7%) verwendet. Die Extinktion betrug bei 590 nm 0,470, entsprechend 0,5402 mg% Hypericin (Extrakt 7).

BEISPIEL 5 a

Es wurde eine Extraktion von Harungablättern (Harunga madagascariensis) durchgeführt. 12,5 g der getrockneten Blätter wurden grob pulverisiert und mit 125 g 1,2-Propandiol versetzt. Die Extraktionsdauer betrug 15 min. bei einer max. Energie von 500 W unter simultaner UV-Licht-Bestrahlung. Es ergab sich eine dunkelrotbraune, wasserlösliche Flüssigkeit mit einer Extinktion bei 590 nm von 1,911, entsprechend 2,1965 mg% Hypericin (Extrakt 8).

BEISPIEL 5 b

Eine Mikrowellenextraktion unter identischen Bedingungen wie in Beispiel 5 a, jedoch ohne UV-Bestrahlung, wies eine wesentlich hellere Farbe als Extrakt 8 auf. Bei 590 nm wurde eine Extinktion von 1,523 gemessen, was einem Hypericin-Gehalt von nur 1,7506 mg% Hypericin entspricht (Extrakt 9).
Es wird abschließend angemerkt, daß beim erfindungsgemäßen Verfahren eine Relation von Droge zu Extraktionsmittel von 20 g/300 g = 1 : 15 bei Johanniskraut vorlag. Bei handelsüblichen Johanniskrautölen, die mit einem Verhältnis von 1 : 4 arbeiten, entsprechen die durchschnittlichen Inhaltsstoffgehalte an Hypericin jedoch nur dem in Beispiel 2 hergestellten Extrakt, so daß durch das erfindungsgemäße Verfahren eine wesentlich höhere Ausbeute des pflanzlichen Ausgangsmaterials möglich ist.

Tabelle 1

| Spektrenvergleiche der in den Beispielen 1 - 5 gewonnenen Extrakte | | | | | |
|---|---|---|---|---|---|
| Bezeichnung: | Verdünnung der Probe: | Lösungsmittel: | Ausgangsmaterial: | E 586-590 Hypericin: | mg% Hypericin: |
| Extrakt 1 | direkt | Olivenöl | Vorgetrocknet | 0,350 | 0,4023 |
| Extrakt 2 | direkt | Olivenöl | Vorgetrocknet | 0,174 | 0,2001 |
| Extrakt 3 | direkt | Olivenöl | Vorgetrocknet | 0,140 | 0,1609 |
| Extrakt 4 | direkt | Olivenöl | Vorgetrocknet | 0,300 | 0,3448 |
| Extrakt 5 | direkt | Miglyol 812® | Vorgetrocknet | 0,431 | 0,4954 |
| Extrakt 6 | direkt | Miglyol 812® | Frisch | 0,497 | 0,5713 |
| Extrakt 7 | direkt | Miglyol 812® | Getrockn. | 0,470 | 0,5402 |
| Extrakt 8 | direkt | Propandiol | Getrockn. | 1,911 | 2,1965 |
| Extrakt 9 | direkt | Propandiol | Getrockn. | 1,523 | 1,7506 |

**Patentansprüche**

1.    Verfahren zur Herstellung von modifizierten Extrakten aus biologischem Material, unter Behandlung des Materials mit Mikrowellen,
dadurch **gekennzeichnet,**
daß das frische oder getrocknete biologische Material zerkleinert und mit einem polaren und/oder apolaren Lösungsmittel versetzt wird und nachfolgend eine Extraktion durch ununterbrochene oder stufenweise Mikrowellenbehandlung zum Aufschluß der Inhaltsstoffe in einem geschlossenen oder offenen Extraktionsbehälter vorgenommen wird, und daß gleichzeitig mit der Extraktionsbehandlung eine die Inhaltsstoffe modifizierende ununterbrochene Bestrahlung durch eine künstliche Strahlungsquelle erfolgt, und das Verfahren fakultativ unter Begasung mit aktiven oder inerten Gasen durchgeführt wird, wobei die Temperatur der Extraktionsmischung bei thermolabilen Inhaltsstoffen zu deren Schutz während des gesamten Verfahrens unter deren Zersetzungspunkt gehalten wird.

2.    Verfahren nach Anspruch 1, bei dem die Leistungsdichte der Mikrowellen im Innenraum des Mikrowellengeräts maximal 42 W/l, vorzugsweise 12 W/l beträgt.

3.    Verfahren nach Anspruch 1 und 2, bei dem als polare Extraktionsmittel Wasser, Alkohole, beispielsweise Ethanol, Methanol, Glyzerin, Propylenglykol, Polyethylenglykol bzw. deren Mischungen eingesetzt werden.

**4.** Verfahren nach Anspruch 1 und 2, bei dem als apolare Extraktionsmittel mikrowellentransparente Mittel, wie Hexan, Dichlormethan, sowie Öle, Wachse und Fette auf pflanzlicher oder Kohlenwasserstoff-Grundlage eingesetzt werden.

**5.** Verfahren nach Anspruch 1 bis 4, bei dem die künstliche Strahlungsquelle eine UV-Lichtquelle ist.

**6.** Verfahren nach Anspruch 1 bis 5, bei dem das biologische Material aus Pflanzen, Pflanzenteilen, Zellkulturen oder deren Mischungen besteht.

**7.** Verfahren nach Anspruch 1, 2, 4 bis 6 bei dem aus frischem oder getrocknetem Johanniskraut durch Mikrowellenextraktion mit mittelkettigen Triglyceriden (Ph.Eur.) bei einer Leistungdichte von ungefähr 12 W/l unter gleichzeitiger Bestrahlung mit einer UV-Lichtquelle hypericinreiches Johanniskrautöl erzeugt wird, wobei die Behandlung mit Mikrowellen stufenweise, die UV-Strahlung ununterbrochen erfolgt.

**8.** Verfahren nach Anspruch 7, bei dem das Verhältnis des pflanzlichen Materials zum Extraktionsmittel 1 : 5 ist und im Extrakt ein Gehalt von mindestens 1 % Hypericin erreicht wird.

**9.** Verfahren nach Anspruch 7, bei dem das Verhältnis des pflanzlichen Materials zum Extraktionsmittel 1 : 15 ist und im Extrakt ein Gehalt von mindestens 0,4% Hypericin erreicht wird.

**10.** Verfahren nach Anspruch 1 bis 3, 5 und 6, bei dem aus getrockneten Harungablättern durch Mikrowellenextraktion mit Propylenglykol (Ph.Eur.) bei einer Leistungsdichte von ungefähr 12 W/l unter gleichzeitiger UV-Lichtbestrahlung hypericinreicher Harungaextrakt erzeugt wird.

**11.** Verfahren nach Anspruch 10, bei dem das Verhältnis des pflanzlichen Materials zum Extraktionsmittel 1 : 10 ist und im Extrakt ein Gehalt von mindestens 2 % Hypericin erreicht wird.

**12.** Vorrichtung zur Durchführung der Verfahren nach Anspruch 1 bis 11, bestehend aus einer Mikrowellenanlage, in der das Extraktionsgut einem geeigneten Gas ausgesetzt werden kann, einem Extraktionsgefäß, das druckfest verschlossen werden kann, einer Strahlungsquelle, die entweder außerhalb oder innerhalb des Extraktionsgefäßes angeordnet ist und einem oder mehreren Thermomeßfühlern, die mit einer Einrichtung zur Regulierung der Temperatur im Inneren der Mikrowellenanlage verbunden sind.

**13.** Extrakte, dadurch gekennzeichnet, daß sie durch ein Verfahren nach einem der Ansprüche 1-11 hergestellt sind.

**14.** Verwendung der Extrakte nach Anspruch 13 in Arzneimitteln, kosmetischen und Körperpflegeprodukten und in Lebensmittelprodukten.

SAMP:        Extrakt 2      REF: Olivenöl

**FIG.1**

SAMP:        Extrakt 3      REF: Olivenöl

**FIG.2**

SAMP: Extrakt 4 REF: Olivenöl

+1.00A

0.200
(A/DIV.)

+0.00A

400.0  100.0 (NM/DIV.)  800.00  NM

**FIG.3**

SAMP: Extrakte 1,4,5 REF: Miglyol®

+1.00A

0.200
(A/DIV.)

5
1
4

+0.00A

500.0  50.0 (NM/DIV.)  700.00  NM

**FIG.4**

SAMP: .  Extrakt 6  Ref: Miglyol®

+2.50A

0.500
(A/DIV.)

+0.00A             NM

400.0   100.0(NM/DIV.)   800.0

**FIG.5**

SAMP:   Extrakt 7  Ref: Miglyol®

+2.50A

0.500
(A/DIV.)

+0.00A             NM

400.0   100.0(NM/DIV.)   800.0

**FIG.6**

10

SAMP:      Extrakte 6 und 7      Ref: Miglyol®

+2.50A

0.500
(A/DIV.)

+0.00A                                          NM

400.0             100.0(NM/DIV.)          800.0

**FIG.7**

SAMP:      Extrakte 8 und 9      Ref: Propandiol

+2.50A

0.500
(A/DIV.)

+0.00A                                          NM

400.0             100.0(NM/DIV.)          800.0

**FIG.8**